# EUROPEAN PATENT SPECIFICATION

(11) **EP 2 728 020 B1**
(45) Date of publication and mention of the grant of the patent: **10.05.2017**
(21) Application number: 11817317.8
(22) Date of filing: 24.11.2011
(51) Int. Cl.: C12P 3/00, B01D 53/62, B01D 53/85, B01D 53/84, C12P 5/02, C12M 1/04, C12M 1/12, C12M 1/107, C12N 1/12, C12M 1/00, C12M 1/42, C12M 1/34

(54) **METHODS AND SYSTEMS FOR THE ABSORPTION OF CO2 AND CONVERSION INTO GASEOUS OXYGEN BY MEANS OF MICROORGANISMS.**
VERFAHREN UND SYSTEME ZUR ABSORPTION VON CO2 UND UMWANDLUNG IN GASFÖRMIGEN SAUERSTOFF ANHAND VON MIKROORGANISMEN
PROCÉDÉS ET SYSTÈMES D'ABSORPTION DE CO2 ET TRANSFORMATION EN OXYGÈNE GAZEUX AU MOYEN DE MICRO-ORGANISMES

(30) Priority: 28.06.2011 ES 201100745
(43) Date of publication of application: 07.05.2014
(73) Proprietor: González Machín, Marcelo Fabián, 35002 Las Palmas de Gran Canaria (ES); Cybel Holding S.A., 1931 Luxembourg (LU)
(72) Inventor: GONZALEZ MACHIN, Marcelo Fabian, 35001 Las Palmas de Gran Canaria (ES)
(74) Representative: Elzaburu S.L.P.
(86) International application number: PCT/ES2011/070813
(87) International publication number: WO 2013/001108

(56) References cited:
- EP-A1- 0 935 991
- WO-A1-2005/001104
- CA-A1- 2 255 287
- CHUL-WOONG CHO ET AL: "Toxicity assessment of common organic solvents using a biosensor based on algal photosynthetic activity measurement", JOURNAL OF APPLIED PHYCOLOGY, KLUWER ACADEMIC PUBLISHERS, DO, vol. 21, no. 6, 3 February 2009 (2009-02-03), pages 683-689, XP019749642, ISSN: 1573-5176, DOI: 10.1007/S10811-009-9401-5
- JEFFREY M GORDON ET AL: "Ultrahigh bioproductivity from algae", APPLIED MICROBIOLOGY AND BIOTECHNOLOGY, SPRINGER, BERLIN, DE, vol. 76, no. 5, 24 July 2007 (2007-07-24), pages 969-975, XP019538763, ISSN: 1432-0614, DOI: 10.1007/S00253-007-1102-X
- ANA P CARVALHO ET AL: "Light requirements in microalgal photobioreactors: an overview of biophotonic aspects", APPLIED MICROBIOLOGY AND BIOTECHNOLOGY, SPRINGER, BERLIN, DE, vol. 89, no. 5, 23 December 2010 (2010-12-23), pages 1275-1288, XP019880861, ISSN: 1432-0614, DOI: 10.1007/S00253-010-3047-8
- JOHAN U GROBBELAAR: "Microalgal biomass production: challenges and realities", PHOTOSYNTHESIS RESEARCH ; OFFICIAL JOURNAL OF THE INTERNATIONAL SOCIETY OF PHOTOSYNTHESIS RESEARCH, SPRINGER, BERLIN, DE, vol. 106, no. 1-2, 26 June 2010 (2010-06-26), pages 135-144, XP019828875, ISSN: 1573-5079

## Description

### Field of the Invention

The present invention relates to methods and systems for the absorption of CO₂ and the conversion thereof into gaseous oxygen by means of microorganisms according to the preamble of Claims 1 and 13.

### Background of the invention

The present invention relates to methods and systems for the absorption of CO₂ and the conversion thereof into gaseous oxygen (O₂) utilising therefor particular microorganisms, such as certain microalgae, spores and manure. Preferably, the CO₂ proceeds from industrial sources, as a result whereof the objective is achieved of reducing atmospheric pollution, generating as a consequence gaseous oxygen beneficial for the environment.

The present invention is based on the observation of the author of the present invention in the sense that, according to diverse publications, certain microorganisms, microalgal biomass, etc, may be utilised having the dual objective of simultaneously absorbing CO₂ and generating lipids employing solely the natural photoperiod and basing the entire invention on the Calvin cycle or photosynthesis. In such cases the cultivation of biomass based on the natural solar light or photosynthetic cycle may confer a certain assurance of success for the production of lipids, together with a satisfactory level of actual absorption of CO₂, being intimately related with the capacity of cells themselves to generate triglycerides, cellular division, and the specific location of the installation, generally based in open pools or ponds or in vertical tubing wherein CO₂ is expelled from the base thereof and wherein a few seconds following the injection thereof a large part of the gaseous mass escapes again to the atmosphere. Nevertheless, if the appropriate conditions could be brought about such that the microorganisms do not lose energy in generating triglycerides but the same is employed in the reproduction thereof leading to an increased rate of growth and reproduction, the absorption of CO₂ and consequent production of O₂ thereby would be substantially increased.

Thus, for example, in the prior art there exist systems based on the cultivation of microalgae for the production of biofuels. All thereof utilise, principally, a single species of microalgae (monoculture) having a very low yield, not permitting resolving the economic equation of the process, and work at temperatures of between 22 °C and 28 °C, operation thereof being restricted to zones having hot climates. On the contrary, the systems and methods of the present invention may be made to function at temperatures lower than 18 °C, in particular between 14 and 16 °C, this having been achieved by acclimatising the microalgae species utilised to such climatological conditions. Moreover, in the systems and methods of the invention appropriate conditions may be selected such that the production of triglycerides is at a minor level or even marginal, substantially orienting the production of the microorganisms towards gaseous O₂.

Moreover, in systems of the prior art, CO₂, and above all industrial CO₂, having an effect on climate change that is becoming more evident every day, is habitually accompanied by other greenhouse gases which are not taken into account in said systems either, which same would severely damage any terrestrial or aquatic biomass cultivation irremediably, as a consequence whereof in the present invention the application thereof has been taken into account very much on an industrial scale, efficiently and consciously based on the multiple disciplines involved and the combination of systems and subsystems rendering it technically viable and scalable.

Utilisation of microalgae for the absorption of CO₂ and the substantial conversion thereof into O₂ has been known for a long time, thus, for example, patent documents US 3 224 143 and US 3 303 608 have already described the conversion of carbon dioxide into oxygen through the use of algae.

More recently, documents WO 92/00380 and US 5 614 378 describe the conversion of CO₂ into O₂ by cyanobacteria when the same are irradiated with radiation having wavelengths between 400 and 700 nm. However, the systems described in said documents are designed for the use thereof in artificial hearts, as a consequence whereof they are not optimised for the production of O₂ on an industrial scale as are those of the present invention, and they lack many of the technical characteristics described in the present invention.

Methods and systems directed towards the conversion of CO₂ into O₂ have also been described in other patent publications, such as EP 0 874 043 A1, EP 0 935 991 A1 and WO 2005/001104 A1, wherein species of Spirulina platensis and of Chlorella vulgaris were utilised as microalgae, and JP 2009007178 A, wherein marine cyanobacteria of the genus Acaryochloris were utilised.

EP 0 939 991 A1 discloses a system for purifying polluted air by using algae such as Spirulina.

In CA 2 255 287 a process of capturing and utilization of carbon dioxide from flue gases is described, wherein the stream of flue gas is passed through a reactor packed with a bed of capturing agent at selected temperatures and pressures, transforming the flue gas in valuable commercial commodities.

Finally, Chul-Woong Cho et al, in "Toxicity assessment of common organic solvents using a biosensor based on algal photosynthetic activity measurement", Journal of Applied Phycol., (2009) 21:683-689 describes that certain common organic solvents, above certain concentration limits, can inhibit algal photosynthesis

Nonetheless, none of said documents describe or suggest methods and systems optimised for the conversion of CO₂ into O₂ presented by the characteristics and advances of the methods and systems of the present invention which, in particular, exhibit notably increased efficiency over those described in the prior art, as shall be described below.

### Summary of the invention

The principal objective sought in the present invention is the conversion of CO₂ into O₂ as a means of providing practical solutions to the latest regulations, laws and measures intended to reduce the carbon footprint to palliate the effects of global warming.

For this purpose, technological solutions have been put into practice utilising species of microalgae different from those customarily utilised in microalgae cultivation systems intended for the obtainment of biofuels, as are the working conditions thereof. The microorganisms utilised in the present invention are different species of microalgae, bacteria and spores which, in perfect symbiosis, act in an efficient manner in the capture of carbon dioxide (CO₂) originating from industrial sources and which, with the possible assistance of tracers based on calcium silicates, convert it into oxygen (O₂). Through the provision of suitable conditions such that the microorganisms practically do not consume energy in generating triglycerides, said energy is employed by the microorganisms in the reproduction thereof, maintaining a maximum rate of growth, finally redounding in a notably increased rate of production of O₂.

A further aspect considered in the present invention is the physical space necessary for the implementation thereof on an industrial scale. The majority of systems require natural light as has already been explained; as a consequence, based on the simple equation of solar irradiation per cm² of surface area, the conclusion is reached that to have the same efficiency as the present invention the systems and methods of the prior art require very extensive land areas which, in the majority of industrial estates, is impracticable or at least economically unviable.

As a consequence, a first aspect of the invention is directed towards a method for purifying contaminated air containing CO₂ by means of the use of microalgae, comprising the steps of:
a) reception of the air containing CO₂ proceeding from a source of contaminated air;
b) physical catalysis, wherein the air containing CO₂ is passed across plates comprising calcium and/or magnesium salts whereon part of the CO₂ of the air becomes fixed in the form of calcium and/or magnesium carbonates;
characterised in that the method moreover comprises the steps of:
c) fermentation, wherein the air proceeding from step b) is passed across a culture comprising a biofamily of microorganisms that comprises microalgae of the classes chlorophyceae cyanophyceae, cryptophyceae, diatomaceae, and/or spores of algae being brown laminariales, red of order gigartinales or green of order ulvales, in any combination thereof, together with at least one organic inhibitor selected from the group consisting of an alcohol, a ketone or a carboxylic acid in any combination thereof, in which step at least part of the remaining CO₂ in the air becomes dissolved in the culture, and;
d) irradiation of the culture with light radiation that is multifrequency and contains from 40 to 60% of radiation having a wavelength of between 400 and 475 nm and an intensity of between 20 W/cm² and 38 W/cm²,
such that photosynthesis is promoted in said microorganisms, causing a reduction in the CO₂ content in the culture through absorption and/or digestion of said CO₂ in the microorganisms and producing O₂.

A second aspect of the invention is directed towards a system for putting into practice the method for purifying contaminated air containing CO₂ through the use of microalgae, comprising the following elements:
a) reception system (1) receiving air containing CO₂ proceeding from a source of contaminated air;
b) plates (2) containing calcium and/or magnesium salts intended to fix part of the CO₂ of the air in the form of calcium and/or magnesium carbonates;
characterised in that the system moreover comprises:
c) tanks (3) for fermentation of the air proceeding from the previous step intended to contain a culture comprising a family of microorganisms that comprises microalgae of the classes chlorophyceae cyanophyceae, cryptophyceae, diatomaceae, and/or spores of algae being brown laminariales, red of order gigartinales or green of order ulvales, in any combination thereof, together with at least one organic inhibitor selected from the group consisting of an alcohol, a ketone or a carboxylic acid in any combination thereof,
wherein such tanks comprise moreover a source of light radiation capable to irradiate the culture with light radiation that is multifrequency and contains from 40 to 60% of radiation having a wavelength of between 400 and 475 nm and an intensity of between 20 W/cm² and 38 W/cm².

The system will be preferably linear in the case wherein the owner thereof desires to generate oxygen gradually. In this manner, if 100 % conversion into oxygen of the gases emitted is not desired from the outset, said value may be attained gradually.

### Brief description of the figure

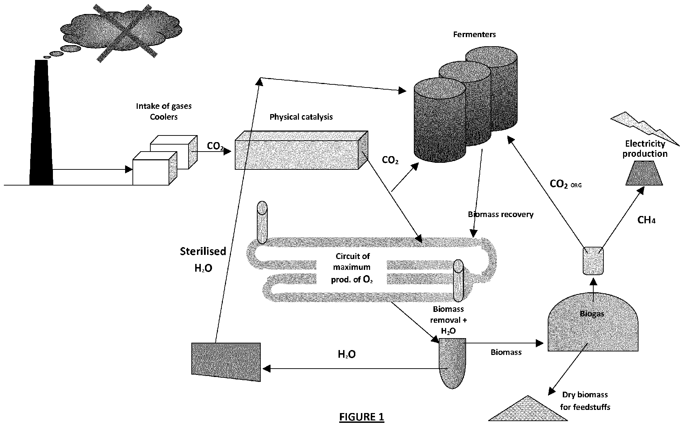

Figure 1 attached shows a general illustrative diagram of a preferred embodiment of the systems and methods of the present invention.

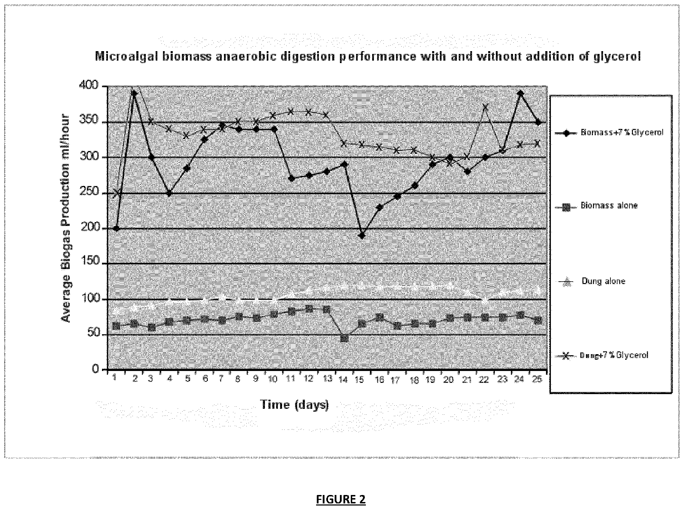

Figure 2 shows the results obtained through the system described in Experimental Example no. 3.

### Detailed description of the invention

The present invention provides systems and methods for the absorption of CO₂, producing gaseous oxygen which may be emitted to the atmosphere, with the objective of reducing emissions of greenhouse gases, principally CO₂ together with methane gas, there moreover having been found the manner of so doing in the minimum space possible, based on artificial photosynthesis and other resources detailed below in the order of the flow diagram.

In a general manner, the culture is developed by providing the microorganisms with carbon dioxide (CO₂), which is mixed and partitioned, adding thereto macro and micro elements, water and tracer elements, being injected in a perfectly proportioned manner together with the CO₂ into blind photobioreactors irradiated with certain wavelengths of the visible spectrum delivered to the microorganisms in the form of photons having the required intensity to obtain a high degree of CO₂ absorption, inhibiting to a great extent the formation of triglycerides, which in other systems are intended for the production of biofuels, the majority being thus converted into O₂.

The systems and methods of the invention provide the possibility of the utilisation of fresh, brackish or salt water which, being utilised in closed circuit, prevents large losses of this resource being produced. Subsequent to taking part in the process the water may be sterilised for reutilisation thereof, to prevent any type of contamination, although the biosystem formed of multiple species of microorganisms substantially inherently prevents the development of other contaminant, competitive or destructive species.

In preferred embodiments, the sea, fresh or brackish water and the CO₂ are treated prior to entry thereof into the system of photobioreactors wherein they are incorporated with the microorganisms and the nutrients required such that, with the incidence of light, growth of the biomass of the system takes place.

The culture system utilised is mixotrophic by virtue of the fact that it is independent of natural light. The species have been acclimatised to receive artificial light of a particular wavelength and defined intensity such that maximum CO₂ capture occurs. Photoautotrophic systems, on the other hand, require natural light for growth of the culture to occur.

Having achieved the planned maximum growth of the biomass, the biomass resulting from the process is deactivated, extracting therefrom biogas through a fermentative oxidation process, utilising therefor natural biological triggers or inducers to accelerate the process (methanogenic bacteria). The composition of the biogas resulting from digestion of the biomass is of approximately 30 - 40 % CO₂ and 60 - 70 % methane. This methane gas (CH₄) obtained may be utilised, for example, for the generation of electricity by means of a turbine. In a particularly preferred embodiment, such turbine is a turbine of the author's design calculated for utilisation with methane (CH₄) and possessing a conversion efficiency of approximately 87 %, differing from modified gasoil or fuel oil generators employing said gas having an approximate conversion efficiency of 50 %. Finally, in preferred embodiments, both the calorific energy together with the low levels of emissions from this process are reutilised and reinjected into the system.

The systems and methods of the invention typically comprise the following steps:
**1 Step of reception of gases:** In general, the majority of large CO₂ emitters realise some type of mitigation of emissions of NOx, SOx and particles utilising diverse filters, catalysts and/or mechanical means, achieving differing degrees of mitigation of emissions, those of CO₂ being the most abundant, expensive and complex to mitigate by virtue of the volume thereof compared with other contaminants.
   In a general manner, the present invention may include a reception or extraction system responsible for extracting or receiving the gases from the source of origin thereof and delivering them under conditions of solution, pressure, temperature and physical precatalysis appropriate for the remainder of the system. In one embodiment, in this reception step the invention possesses a cooling prechamber for the gases wherein they are received from the industrial installations of origin thereof through a simple conventional industrial extractor prepared for high temperatures. Should it be present, said extractor will be dimensioned in accordance with the volume of gas which the owner of each plant desires to be absorbed, and the precooling may be realised by any method known to a person skilled in the art.
   In another particularly preferred embodiment, the reception system of gases comprises a circuit containing a particular solvent in circulation, wherein both the CO₂ and the other accompanying gases, such as NOx, SOx, etc, to mention some thereof, may be dissolved, buffered, precatalysed and/or cooled to the appropriate temperature, as may be deemed necessary or suitable, prior to entering the remainder of the system.
**2 Step of physical catalysis:** Having been appropriately cooled the gases proceeding from the foregoing step are passed to a step of physical catalysis. In a preferred embodiment this step is realised within a chamber having a humidity of from 40 % to 90 %, preferably 80 % relative humidity, or a series of similar chambers wherein basically the cooled gases are made to circulate past catalytic plates placed in a horizontal or vertical orientation according to the space available. As will be seen below, the entire system may be located underground, principally by virtue of it being based on photosynthesis generated by artificial light, as a result whereof the visual pollution caused by systems found habitually in the market is moreover avoided.

Said plates have been designed and developed for the present invention starting with generally very abundant materials preferably originating from urban waste, principally calcium or magnesium silicates, bonding them in such manner that they present sufficient porosity to permit the passage of gases, particularly the gaseous CO₂ and the CO₂ dissolved in water. For this purpose, in a preferred embodiment, at the time of manufacture thereof and prior to the setting thereof, the plates are quickly pierced by needles of differing diameters, leaving therein a multiplicity of orifices piercing them diametrically, for example hundreds thereof, permitting an increase in the permeability thereof or, what is the same thing, the velocity of mineral carbonation in the same manner wherein CO₂ in nature reacts with non-carbonated minerals to form carbonates, which reactions in nature are normally slow; this constitutes a first barrier of secure fixing of CO₂ or oxygen generation. In a particularly preferred embodiment, said plates contain the following materials in the following proportions:
- 10 % to 30 % CaO;
- 5 % to 25 % calcium carbide (CaC₂);
- 15 to 25 % calcium hydroxide Ca(OH)₂;
- 10 to 50 % calcium carbonate (CaCO₃);
- 5 % to 50 % magnesium (Mg);
- 5 % to 15 % aluminium filings (Al).

In a particularly preferred embodiment, 50 % of the aforementioned calcium carbonate is obtained from the finely ground shells of oysters, clams and scallops obtained from restaurants and seafood processing plants and, to a lesser degree, of those of mussels, which otherwise would end up in dumps, signifying a manner of increasing the value of waste which otherwise might become contaminants. The general reaction which occurs, as in nature, is exemplified in the following manner:

(*Mg,Ca*)*ₓSi_{y}O_{x+2y+z}* + *xCO₂ → x(Mg,Ca*)*CO₃* + *ySiO*₂ + *zH₂O*

Normally the percentage of finely ground calcium and magnesium silicates incorporated into the closed circuit of extraction of gases lies between 20g/l and 250 g/l.

In particular locations, such as in Chubut, Argentina, wherefrom the inventor originates, high percentages of olivine have been found in local clayey soils which may also be utilised in different proportions in the aforementioned formulation, it having been observed that one tonne of pure olivine may achieve the storage (crushed) of up to 2.3 tonnes of CO₂ in a relatively short time.
Olivine from Chubut:

   Mg₂SiO₄ + 2CO₂ → 2MgCO₃ + SiO₂

In the system described, and by virtue of the fact that normally sequestration of CO₂ from chimneys is undertaken through closed circuit scrubbing by water stream (physical catalysis module - chimney - physical catalysis module loop) also rich in silicates, consequently part of the fixing of CO₂ already occurs through the very dilution of the CO₂ in water and in permanent contact within this loop.

Said system, which furthermore may serve to cool the inlet gases which, in most plants, range between 130 °C and 600 °C in many cases, will generate steam pressure in the circuit and will raise the temperature in the first absorption module which will operate, depending on the plant in question in respect of the installation of this system, at a temperature of between 100 and 200 °C and under a regime which will increase the permeability of the plates and, as a consequence, the speed of CO₂ fixing. This module is made to work as a cascade condenser, balancing the pressures and extracting the CO₂ at a lower temperature by means of a vacuum towards a second optional physical catalysis module which will preferably work at lower temperatures, of the order of 21 °C, and 1 atm pressure.

The original principal reactants (CO₂ and silicates) when mutually combined experience a reduction in the volume thereof, carbonates being some 900 times more dense (weighted average among those referred to) than CO₂ in gaseous state at approximately 20 °C and 1 atm. On the CO₂ being fixed in the minerals (solid phases) of the catalytic plates, that is to say the CO₂ having been incorporated into the solid phase, the assembly (depending on the mixtures and the percentages thereof) generates increases in weight and volume ranging from 10 % to 150 %. The panels, having attained saturation, may be exchanged and easily utilised by different types of plant, especially cement plants. Moreover, as already stated, the raw materials are abundant and may be obtained from different places at very competitive price and having become saturated they are 100 % recyclable without any type of special treatment other than the grinding thereof once more.

Preferably, said plates are periodically analysed to evaluate the constitution and catalytic efficiency thereof. The most convenient standard dimensions of the module are 2.25 m wide x 2.50 m high x 12.5 m long in order that they may be rapidly exchanged by lorry and transported to special reassembly installations. Moreover, the new units may be interconnected such that it is not necessary to stop the cycle to change the plates of the integral oxygen production circuit. Diverse chemical reactions being realised in the catalysis, the containers therefor are preferably plastic coated internally and the floors thereof are installed in the form of trays to contain and recirculate the different elements of the plates which decompose and drip as a result of to the high humidity of the environment and acidification of the medium caused by the CO₂.

These modules will be preferably dimensioned to substantially absorb the remains of the NOx and SOx of the inlet gas, together with from 20 % to 25 % of the CO₂ contained therein, which the materials described in the composition of said plates will catalyse and/or convert into O₂. The 75 to 80 % balance of CO₂ gas, although still containing minimal traces of NOx and SOx, will be sent to the subsequent fermentation step.

**3 Fermentation step:** The gases proceeding from the foregoing step are transferred to fermenter tanks wherein they are passed across a culture containing a biofamily of microorganisms of the type of especially acclimatised microalgae and spores having great capacity for the absorption or biofixation of CO₂ and liberation of oxygen. In a preferred embodiment, such biofamily of microorganisms comprises any subset of the following species:
Clorophyceae: Chlorella vulgaris, Chlorella saccharophila, Lobomonas *sp*, Scenedesmus acuminatus, Scenedesmus quadricauda, Scenedesmus *sp*, Scenedesmus desmodesmus, Ankistrodesmus angustus, Monoraphidium griffithii, Elakatothrix gelatinosa, Golenkinia radiata, Dictyosphaerium pulchellum, Sphaerocystis schroeteri, Oocystis *sp,* Selenodyctium brasiliensis,
Cyanophyceae: Chroococcus *sp*, Cyanophyceae filamentosa, Arthrospira platensis, Arthrospira maxima, Nostoc *sp*, Nostoc ellipsosporum, Nostoc spongiaeforme, Anabaena macrospora, Anabaena monticulosa, Anabaena azollae, Spirulina platensis, Spirulina maxima, Spirulina orovilca, Spirulina jeejibai, Spirulina lonar, Prorocentrum dentatum, Noctiluca scientillans, Trichodesmium sp., Aurantiochytrium,
Cryptophyceae: Cryptomonas *sp,* Cryptomonas brasiliensis;
Diatomaceae: Centrica (unidentified), Nitzschia *sp,* Skeletonema costatum,
Spores and manure: Brown Laminariales (Macrocystis pyrifera, Undaria pinnatifida); Red of Order Gigartinales (Gigartina skottsbergii, Kappaphycus alvarezii), Green of Order Ulvales (Enteromorpha prolifera).

All these species, in the form of an assembly thereof (biofamily) or separately or in any combinations and proportions of natural or induced predominance thereof, may be developed in fresh, brackish or sea water in appropriate proportions of dilution and temperature according to the region wherein the cultivation thereof is established, or they may be cultivated in water or in an artificial cultivation medium having the following characteristics, according to a preferred embodiment:
For every 1000 ml of distilled or bidistilled water there will be artificially added the following elements, ensuring very suitable performance of the species stated for the objective sought, the same being capable of being developed separately or as an assembly (biofamily), it being possible to regulate the proportions in the sense of the minimum percentages stated (less brackish to fresh water) or the maximum percentages stated (less brackish to sea water, including having salt lake characteristics):

| | |
|---|---|
| NaCl | From 3 to 33 g/l, preferably approximately 11 g/l; |
| KCl | From 0.1 to 0.9 g/l, preferably approximately 0.4 g/l; |
| MgSO₄ | From 1 to 3 g/l, preferably approximately 1.50 g/l; |
| Na₂SiO₃·9H₂O | From 0.1 to 0.9 g/l, preferably approximately 0.5 g/l; |
| FeSO₄·7H₂O | From 1 to 8 mg/l, preferably approximately 3 mg/l; |
| Na₂EDTA | From 1 to 9.6 mg/l, preferably approximately 2.7 mg/l; |
| CaCl₂ | From 0.1 to 0.25 g/l, preferably approximately 0.10 g/l; |
| MnCl₂·4H₂O | From 1 to 5 g/l, preferably approximately 2 g/l; |
| CoCl₂ | From 1 to 9 µg/l, preferably approximately 2.3 µg/l; |
| CuCl₂·2H₂O | From 1 to 20 µg/l, preferably approximately 15 µg/l; |
| ZnCl₂ | From 0.1 to 0.7 mg/l, preferably approximately 0.3 mg/l; |
| H₃BO₃ | From 20 to 40 mg/l, preferably approximately 31.5 mg/l. |

The present invention is based on observations realised by the author hereof on the occasion of the construction of a new pool for the realisation of experiments within the project giving rise to the present invention. The new pool was constructed in concrete, however for the use thereof in the experimental tests it required to be plastic-coated externally, for which purpose there was applied to the concrete a layer of paint known as gel coat and requiring dilution with acetone and cutting with paraffin. In hot climates complete curing of said paint may occur in 7 days however, in the climate of Chubut, Argentina, curing thereof was prolonged for a very much longer time. As subsequently proven, this caused the realisation of the first experimental tests carried out with active biomass under real conditions to commence without the material having fully cured and, as a consequence thereof, in the inadvertent presence of acetone in the pool and, consequently, in the composition of the culture. Under these conditions it was observed that whilst in previous experimental tests the active biomass had demonstrated rates of fixing of triglycerides in cells exceeding 14 %, however, in the pool containing acetone, over the course of 10 days not only had the level of triglycerides not increased but, on the contrary, it had decreased.

Based on these observations, experiments were carried out with diverse combinations with acetone and, furthermore, with other ketones, alcohols and organic acids, some thereof yielding excellent results as inhibitors of the production of triglycerides by cells. This redounded to the benefit of the efficiency demonstrated by the biofamily by virtue of the fact that, according to what is believed, the slight but continuous stress to which the cells were subsequently subjected in the circuit of maximum production of oxygen caused therein an increase in the requirement thereof for CO₂ consumption, permitting the establishment of very long photoperiods of up to 19 hours without the cells entering a photoinhibition phase and without affecting the reproduction thereof.

Consequently, from the tests realised, it was possible to conclude that when there is present in the culture at least one organic inhibitor selected from the group comprising an alcohol, a ketone or a carboxylic acid, and in a more preferred manner ethanol, acetone or propanoic acid and/or pentanoic acid, and on then irradiating the said microorganisms with light radiation of a particular frequency, intensity and duration, by means of a mechanism which has been established although still not fully understood, the said microorganisms develop inhibition towards intra- and extracellular triglyceride fixation, leading to high metabolic activity and a requirement for carbon, this being translated into high consumption of CO₂ and emission of O₂. In a preferred embodiment, the light radiation applied to the microorganisms is multifrequency, however containing from 40 to 60 %, and preferably approximately 50 %, blue light having a wavelength of between 400 and 475 nm, the other wavelengths of the visible spectrum, such as red, yellow, etc, optionally excluding green, preferably participating in the remainder of the light radiation, all thereof having an intensity of at least 20 W/cm² to 38 W/cm².

Furthermore, the author hereof observed that, in the irradiation step, if the microorganisms were additionally irradiated for 3 seconds every minute at an intensity of between 5 and 15 W/cm² with ultraviolet light having a wavelength of between 400 and 200 nm without exceeding an energy of 3·10⁶ eV per photon, preferably in combination with the aforementioned organic inhibitor, destruction of the DNA of the cyanobacteria does not occur nor is the regime of photoinhibition thereof reached, but nevertheless the same are induced to produce up to 2.5 kg of oxygen per 2.8 kg of CO₂ provided to each kilogram of biomass.

A further characteristic of the systems and methods described, differing from other systems of the prior art, is that the biofamily of microorganisms multiplies very adequately in the described percentages at water temperatures of between 14 °C and 18 °C, in comparison with other systems only operating above 22 degrees Celsius and up to 28 °C. The inventor hereof has calculated that the main body of heavy industry worldwide (exceeding 60 %) is located above the Tropic of Cancer, that is to say, in cold climates. Consequently, a system such as that of the present invention, being energetically balanced and achieving the purpose for which it is designed, will preferably comprise microalgae species which function optimally in cold climates.

The system of the invention is furthermore more efficient in the use of energy by virtue of the fact that it is possible to take advantage of the residual heat of the exchangers at the beginning of the system.

In order that the biofamily of microorganisms realises its function adequately, it is advantageous that suitable nutrients be made available in the culture. In a preferred embodiment, the formulation of the nutrients, arising from the study of the impact of the nitrogen cycle in nature and the different alterations thereto through human activities, is that stated below, wherein the percentages quoted are provided weekly (between 7 and 9 days) and refer to percentages by weight of the quantity of biomass resident in the system (alive):
Gaseous nitrogen (N₂): between 1 % and 30 %, preferably approximately 15 %;
Nitric acid: between 1 % and 30 %, preferably approximately 7 %;
Ammonium chloride (NH₄Cl): between 1 % and 30 %, preferably approximately 7.5 %;
Phosphorus oxide (P₂O₅), from 1 % to 30 %;
Ammonium nitrate (NH₄NO₃): between 1 % and 30 %, preferably approximately 13 %;
Potassium oxide (K₂O): between 1 % and 40 %, preferably approximately 23 %;
Magnesium oxide (MgO): between 1 % and 30 %, preferably approximately 5 %;
Sulphur trioxide (SO₃): between 1 % and 40 %, preferably approximately 23 %;
Calcium oxide (CaO): between 1 % and 50 %, preferably approximately 13 %;
Total boron (B): 0.05 %, between 0.01 % and 5 %;
Total iron (Fe): 0.07 %, between 0.01 % and 7 %;
Total zinc (Zn): 0.05 %, between 0.01 % and 30 %;
the remainder of the culture being water, which may be fresh, brackish or salt.

In this culture the biomass has a concentration of 1 to 100 g/l, preferably approximately 27 g/l. Moreover, in the base of the fermenter tank there is preferably located a distributor promoting the dissolution of nutrients together with the dissolution and disaggregation of microscopic bubbles of CO₂, which amply facilitates absorption by the biosystem and biofamily comprising it during the artificial light photoperiod of from 14 to 18 hours. The remainder of the time, in the dark phase, atmospheric air is provided to it which, as is known, possesses a high N₂ content, which will also be present and dissolved in the water and available for the illumination phase.

**4 Circuit of Maximum Production of O₂:** In an optional embodiment, the culture is subsequently subjected to a step of maximum production of O₂. The underlying idea of this step of the process arose through the inventor, of Patagonian origin, identifying that the majority of the light received by the family of microorganisms utilised, originating from typically Patagonian species, was basically blue light (photosynthesis in the abyss) in the natural habitat thereof in Patagonia at a depth of 21 m and a temperature of 8 °C. Nevertheless, due to the range of high and low tides, as the tide started to go down said microalgae were gradually irradiated with different light frequencies determined by the depth of the water at each moment. This, according to what is believed, has led to the microalgae biofamily utilised having become accustomed to being selective of particular light frequencies, absorbing more efficiently light at frequencies around light which is bluish. This gave rise to the idea of providing these microalgae with light irradiation of specific wavelengths around those whereto they have naturally acclimatised, although at double or greater light intensity, without substantial photoinhibition being observed therein. With this reasoning, the author of the present invention developed a photoperiod of 14 to 18 hours of artificial light having an intensity and weighted light dispersion exceeding twice that which the microorganisms would receive under natural conditions, generating exponential growth thereof, extremely high CO₂ absorption and O₂ production being a consequence thereof.

The foregoing observations have been manifested in an experimental circuit being conveniently a blind photobioreactor by virtue of the fact that the walls thereof do not require to be translucent. Consequently, it may be constructed of metal, PVC plastic or nylon, according to the conditions of location and climatology. According to the quantity of oxygen to be produced, the diameter of said tubes will be calculated for a given quantity of biomass and light radiation of irradiated light by given sector and circuit. Not all tubes require to have the same diameter by virtue of the fact that Bernoulli's principle is utilised to achieve maximum energy savings in impulsion and recirculation, together with subjecting the culture present in the circuit to the desired pressure at each moment, as a result of appropriate selection of the tubing diameter at each point. Moreover, in a particularly preferred embodiment, strips of LED diodes, optical fibre or organic LEDs will be disposed in the interior of the tube to ensure the Calvin cycle during a photoperiod irregular in light frequency and intensity. That is to say, the circuit is divided into sections wherein the biomass dwells during a given period, and in each thereof the biomass will be subjected to a particular pressure and irradiated with a particular irradiation. In agreement with a general illustrative scheme of the conditions of pressure, irradiation and period of residence of the microorganisms in each section of the module of maximum production of O₂, according to a preferred embodiment, the circuit will be divided into the following sections:
- Section 1: Tubing of 25 to 100 mm in diameter. In this section the culture will be irradiated with light radiation having frequencies of between 400 and 520 nm at an irradiation intensity of between 30 and 50 W/cm² and will be subjected to a pressure of from 1.8 to 5.5 atm, remaining in the same for a period of between 10 minutes and 24 hours;
- Section 2: Tubing of 63 mm to 120 mm in diameter. In this section the culture will be irradiated with light radiation having frequencies of between 521 and 580 nm at an irradiation intensity of between 10 and 20 W/cm² and will be subjected to a pressure of from 1.0 to 1.79 atm, remaining in the same for a period of between 3 minutes and 24 hours;
- Section 3: Tubing of 83 mm to 180 mm in diameter. In this section the culture will be irradiated with light radiation having frequencies of between 581 and 620 nm at an irradiation intensity of between 21 and 31 W/cm² and will be subjected to a pressure of from 0.5 to 1.25 atm, remaining in the same for a period of between 3 minutes and 24 hours;
- Section 4: Tubing of 181 mm to 750 mm in diameter. In this section the culture will be irradiated with light radiation having frequencies of between 621 and 750 nm at an irradiation intensity of between 30 and 5 W/cm² and will be subjected to a pressure of from 0.01 to 1.249 atm, remaining in the same for a period of between 1 minute and 24 hours.

Thus, for example, in an experimental practical embodiment, the biomass commenced being irradiated with an irradiation of between 5 W and 50 W of violet light, the biomass having a concentration of 36 g of biomass per litre of water and being displaced within the tube at a speed of between 1 km/h and 10 km/h, such that the first litre of water entering the tube returned through the other extremity 24 hours later. In this trajectory of 24 hours the different light frequencies succeeded one another, in this manner running through the light spectrum, commencing with violet light and continuing through blue, green, yellow, orange and red, to end with violet again. Between the change from one colour to another there were introduced sections of darkness wherein the microorganisms were maintained in darkness for a particular period of time, for example half an hour. In this manner the biomass will pass from a photoautotrophic state, to a heterotrophic state and to a mixotrophic state.

In a particularly preferred embodiment, through the interior of the tubes of the circuit run hoses of LEDs, organic LEDs or optical fibre maintained centred within the same by a system of double rings similar to the Mercedes Benz emblem, through the centre whereof passes a ring having bristle brushes supporting the lights, the exterior ring thereof making contact with the interior walls of the tube. All thereof are joined to one another by a thin steel cable, connected at the extremities of each tube, to an external rotary roller which receives all thereof, passing through the same number of glands preventing water from escaping. Once per month the roller, which has attached thereto a pinion and a low speed electric motor, winds the cables according to the roller of the desired extremity, causing the displacement of all rings towards the same which by means of their brushes clean the adhered biomass from both the line of lights and the exterior of the tube. In other types of photobioreactor of the prior art, after a time the surfaces thereof become saturated with biomass and manure and the efficiency thereof is reduced by up to 90 %. At the end of the circuit the oxygen produced is vented.

According to a preferred embodiment, every 7 days a certain percentage of the biomass extracted is removed by cavitation to pass to the methanisation tanks. In this case new biomass is added to the fermenters in the same percentage. The entire circuit of lights preferably has a voltage of 12 V or 24 V, consuming less than 2 W per tonne of oxygen produced and which may be easily supplied by alternative energy, whether wind, photovoltaic, methane, minihydraulic, etc, if the conditions of the location so permit. In this manner a minimum energy balance is maintained for a highly efficient system.

The composition of the resultant dry biomass will depend on the characteristics of the system, on the geographical factors of the location of installation of the system, and on the multiple species utilised. A standard composition per gram of biomass having up to a maximum of 4 % humidity would be the following:
Protein: 56 - 71 %
Carbohydrates: 10 - 17 %
Lipids: 6 - 14 %
Nucleic acids: 1 - 4 %
Beta-carotenes and Omegas 3, 6 and 9.

**5 Biomass methanisation or deactivation tank:** The biomass removed from the previous step is then optionally transferred to a tank or tanks for such purpose, which may be implemented in the form of what are known as biogas plants, having the objective of proceeding to the methanisation or deactivation of the biomass. In the equivalent systems of the prior art, to deactivate a given biomass requires a period of 10 to 12 days. On the contrary, in the present invention, by virtue of the fact that this system utilises a growth trigger for the microorganisms and more efficient and rapid deactivation/methane production, deactivation is achieved in an average of 7 days under any conditions of temperature and with any biomass. The trigger is basically organic glycerine, a very abundant and low-cost byproduct generated, for example, through the production of biodiesel. Being biodegradable it is perfectly assimilable into animal feedstuff. In this manner the biomass is deactivated preventing the methane being slowly emitted to the atmosphere, by virtue of the fact that otherwise the system would be negative in oxygen production given that 1 tonne of methane is equivalent to 21 tonnes of CO₂. If one tonne of plant or microalgae biomass produces a weighted average of 5500 litres of methane per day on adding a grade C glycerine trigger, daily production increases by 32 %, however it is deactivated beforehand. That is to say that the presence of the trigger does not make the microorganisms extract more methane from a given biomass, what it does is to extract it in less time.

From this methanisation leaves a biogas which in fact is of high purity, being 30 % CO₂, sent to a separator, there being obtained 30 % organic CO₂ which is sent to the fermenters and 70 % methane (CH₄), which is sent to an electricity generating turbine especially designed solely for use with CH₄ having a conversion efficiency exceeding 81 % and very low indices of emissions, which are reinjected into the initial module of intake of gases.

In this manner, from 100 % of CO₂ emissions the conversion is achieved of 40 % to 60 % O₂, in addition to abundant electricity from a sustainable source. This sustainable source is obtained from producing and burning the methane (CH₄), byproduct of the fermentation or deactivation of the biomass, prior to the conversion thereof into algae meal or algae flour.

**6 Water treatment plant:** Finally, the water having been separated from the biomass in the cavitation tank, it is optionally treated in a treatment and sterilisation plant based on the physical treatment thereof without the involvement of chemical products. Having been suitably treated it is possible to reinject it into the circuit of the fermenters.

In general, in the methods and systems of the invention natural light may be combined with artificial light in any proportion. Moreover, a fixed or variable photoperiod may be utilised, the latter option being of particular interest at sites having very marked seasons with different climates and light amplitudes. Furthermore, the fermentation tanks may optionally be installed having a particular angle with respect to the ground, for example at 45°, and having a particular orientation to achieve better solar tracking and, as a consequence, an additional increment in photosynthetic efficiency.

### Experimental Examples

### EXAMPLE 1

In an illustrative experimental realisation, the fermenters were cylindrical tanks having a central light and therein the water for cultivation and growth of this biofamily was made to slowly rotate at 6 rpm. The culture containing the said biofamily of microorganisms had a concentration of 42 g/l of live biomass and was irradiated during a photoperiod of 14 hours per day on average with blue light of wavelength from 400 to 475 nm and with an intensity of at least 20 W/cm² to 38 W/cm². For each tonne of active biomass in the circuit there were injected thereinto, once every 30 to 45 days, the following organic inhibitors in the quantities stated:
1. From 500 ml to 7500 ml, preferably 5000 ml, of acetone;
2. From 100 ml to 5000 ml, preferably 3500 ml, of propanoic acid;
3. From 300 ml to 10 000 ml, preferably 7500 ml, of pentanoic acid;
4. From 1000 ml to 5000 ml, preferably 3000 ml, of ethanol.

As a result, the rate of growth of the biomass in all cases was greater than or equal to 8 % per day of the initial quantity on installation of the circuit, whilst the total production of triglycerides calculated at the end of the experiment was only between 3 and 5 % by weight with respect to the weight of the biomass. In similar experiments carried out in the absence of inhibitor the total production of triglycerides was approximately 14 %, demonstrating the benefit obtained through the use of the organic inhibitor in the culture.

In a further embodiment of this practical experiment, the maximum dwell time of the biomass in the fermenter was between 4 and 6 days, a variable percentage of between 10 and 40 % of said biomass being removed every 5 days on average in order to inject it into the principal absorption circuit. That is to say, the fermenters are used in this mode for the superintensive growing of biomass which may be optionally transferred to the principal absorption circuit to replace that which may be lost through mitochondrial overexcitation and profound photoinhibition. That is to say, it takes the role of what would be a seedbed in traditional agriculture.

### EXAMPLE 2

In a further experimental example carried out under the same conditions as in Example 1 and with acetone as organic inhibitor, over 10 days there was obtained a total of 600 kg of biomass, there being removed a weighted average of 1.8 kg of triglycerides per kg of biomass, or 1.8 %. Subsequently, the use of inhibitor was ceased, adding water to the pool at a rate of 10 % per day. After 7 days samples of biomass were taken at different points of the pool until making up a total of 1 kg of extracted sample. This sample was subjected to extraction by steam distillation (selective distillation), there being obtained 8.3 % of triglycerides of the total thereof compared with the previous 1.8 %, thus demonstrating the efficiency of the inhibitor employed.

### EXAMPLE 3: Glycerol as trigger of the process of methanisation of the biomass

Additionally, the effect was tested, in the methanisation step of the biomass, of the addition of 7 % glycerol as trigger of the process. Said glycerol, dissolved in the process water itself, was added at the inlet to the methane tank. The results are shown in Figure 4 attached, from which may be clearly deduced the multiplication effect produced by the provision of glycerol on the average production of biogas in ml/hour.

This technology is applicable to any source of emission of CO₂, although it is particularly suitable for highly contaminant sources such as cement, petrochemical, steel, petroleum and electricity generation plants, furthermore the versatility thereof permits the utilisation thereof in cities, motorways or tunnels, wherein it may capture a wide variety of gases.

## Claims

1. Method for the purification of contaminated air containing CO₂ by means of microorganisms, comprising the steps of:
a) reception of air containing CO₂ proceeding from a source of contaminated air;
b) physical catalysis, wherein the air containing CO₂ is passed across plates comprising calcium and/or magnesium salts, whereon part of the CO₂ of the air becomes fixed in the form of calcium and/or magnesium carbonates;
**characterised in that** the method further comprises the steps of:
c) fermentation, wherein the air proceeding from step b) is passed across a culture comprising a biofamily of microorganisms that comprises microalgae of the classes chlorophyceae cyanophyceae, cryptophyceae, diatomaceae, and/or spores of algae being brown laminariales, red of order gigartinales or green of order ulvales, in any combination thereof, as well as at least one organic inhibitor selected from the group consisting of an alcohol, a ketone or a carboxylic acid in any combination thereof, in which step at least part of the remaining CO₂ in the air becomes dissolved in the culture; and irradiation of said culture with light radiation that is multifrequency and contains from 40 to 60% of radiation having a wavelength of between 400 and 475 nm and an intensity of between 20 W/cm² and 38 W/cm²,
such that photosynthesis is promoted in said microorganisms, causing a reduction in the CO₂ content in the culture through absorption and/or digestion of said CO₂ in the microorganisms and producing O₂.

2. Method according to Claim 1 wherein the organic inhibitor of step c) is ethanol, acetone, propanoic acid or pentanoic acid in any combination thereof.

3. Method according to Claim 2 wherein the organic inhibitor of step c) is acetone.

4. Method according to Claim 1 wherein the light radiation applied to the microorganisms contains approximately 50% of radiation having a wavelength of between 400 and 475 nm and an intensity of between 20 W/cm² and 38 W/cm².

5. Method according to any one of Claims 1 to 4 wherein in step c) for 3 seconds every minute the microorganisms are furthermore irradiated with additional light radiation having a wavelength of approximately 200 nm and an intensity of between 5 and 15 W/cm², without exceeding an energy of 3·10⁶ eV per photon.

6. Method according to any one of the foregoing claims wherein the dwell time of the microorganisms in step c) is from 4 to 6 days, and every 5 days there is removed therefrom a percentage varying from 10 % to 40 % of the microorganisms.

7. Method according to any of the foregoing claims wherein, following step c), the following additional step is applied:
d) maximum production step of O₂, wherein the culture from step c) is passed along a circuit wherein it is subjected to a series of pressures in succession lying between 0.01 atm and 5.5 atm and to irradiation with a series of frequencies of the light spectrum passing through violet, blue, green, yellow, orange and red, returning to violet also in succession, the radiation having an intensity of between 5 and 50 W/cm², thereby causing an additional reduction in the CO₂ content in the culture through absorption and/or digestion of said CO₂ in the microorganisms and producing O₂.

8. Method according to Claim 7 wherein the culture is subjected to the following sequence of light radiation:
- a first section wherein the culture is irradiated with light radiation having frequencies of between 400 and 520 nm at an irradiation intensity of between 30 and 50 W/cm² and is subjected to a pressure of from 1.8 to 5.5 atm, remaining in the same for a period of between 10 minutes and 24 hours;
- a second section wherein the culture is irradiated with light radiation having frequencies of between 521 and 580 nm at an irradiation intensity of between 10 and 20 W/cm² and is subjected to a pressure of from 1.0 to 1.79 atm, remaining in the same for a period of between 3 minutes and 24 hours;
- a third section wherein the culture is irradiated with light radiation having frequencies of between 581 and 620 nm at an irradiation intensity of between 21 and 31 W/cm² and is subjected to a pressure of from 0.5 to 1.25 atm, remaining in the same for a period of between 3 minutes and 24 hours;
- a fourth section wherein the culture is irradiated with light radiation having frequencies of between 621 and 750 nm at an irradiation intensity of between 30 and 5 W/cm² and is subjected to a pressure of from 0.01 to 1.249 atm, remaining in the same for a period of between 1 minute and 24 hours.

9. Method according to Claim 8 wherein between each section and that following the culture is subjected to a period of darkness wherein it is not irradiated.

10. Method according to any of foregoing Claims 8 or 9 wherein the sequence of irradiations is applied to the culture in photoperiods of from 14 to 18 hours per day.

11. Method according to any of the foregoing claims wherein every particular number of days at least a part of the microorganisms is removed from the circuit of maximum production of O₂ and transferred to an additional step of:
e) methanisation, wherein the microorganisms are deactivated by means of a procedure of fermentative oxidation, there being obtained a deactivated biomass mixed with water and a biogas comprising CO₂ and methane.

12. Method according to Claim 11 wherein fermentative oxidation is carried out utilising an inducer accelerating the process, said inducer being glycerol.

13. System to purify contaminated air containing CO₂ by means of microorganisms comprising the following elements:
a) reception systems (1) receiving air containing CO₂ proceeding from a source of contaminated air;
b) system of plates (2) containing calcium and/or magnesium salts intended to fix part of the CO₂ of the air in the form of calcium and/or magnesium carbonates;
**characterised in that** the system further comprises:
c) tanks (3) for fermentation of the air proceeding from the previous step intended to contain a culture comprising a family of microorganisms that comprises microalgae of the classes chlorophyceae, cyanophyceae, cryptophyceae, diatomaceae, and/or spores of algae being brown laminariales, red of order gigartinales or green of order ulvales, in any combination thereof, as well as at least one organic inhibitor selected from the group consisting of an alcohol, a ketone or a carboxylic acid in any combination thereof,
wherein such tanks further comprise a source of light radiation capable to irradiate the culture with light radiation of the visible light spectrum that is multifrequency and contains from 40 to 60% of radiation having a wavelength of between 400 and 475 nm and an intensity of between 20 W/cm² and 38 W/cm².

14. System according to Claim 13 furthermore comprising circuit (4) of maximum production of O₂, including an assembly of tubing intended for the passage therethrough of the culture from fermentation tanks (3), which tubing comprises tubes of different diameters ranging between 25 and 750 mm capable of subjecting the culture to a particular sequence of pressures of between 0.01 atm and 5.5 atm, and a source of light radiation capable of irradiating the culture with a particular sequence of frequencies of the light spectrum passing through violet, blue, green, yellow, orange and red, returning again to violet, with an irradiation intensity of between 5 and 50 W/cm², simultaneously.

15. System according to any one of the foregoing Claims 13 or 14 further comprising methanisation tanks (5) intended to deactivate the microorganisms by means of a procedure of fermentative oxidation, there being obtained therefrom a deactivated biomass mixed with water and a biogas comprising CO₂ and methane.

## Patentansprüche

1. Verfahren zur Reinigung kontaminierter, CO₂-haltiger Luft durch Mikroorganismen, umfassend:
a) Empfang CO₂-haltiger Luft von einer Quelle kontaminierter Luft;
b) physikalische Katalyse, wobei die CO₂-haltige Luft über Platten geleitet wird, die Calcium- und/oder Magnesiumsalze enthalten, wobei ein Teil der CO₂-haltigen Luft in Form von Calcium- und/oder Magnesiumcarbonat gebunden wird;
**dadurch gekennzeichnet, dass** das Verfahren weiterhin die folgenden Schritte umfasst:
c) Fermentation, wobei die Luft aus dem Schritt b) über eine Kultur geleitet wird, die eine Bio-Familie aus Mikroorganismen umfasst, die Mikroalgen der Klassen Chlorophyceae, Cyanophyceae, Cryptophyceae, Diatomaceae und/oder Sporen von Braunalgen der Ordnung Laminariales, Rotalgen der Ordnung Gigartinales oder Grünalgen der Ordnung Ulvales in jeder Kombination derselben sowie mindestens einen organischen Inhibitor umfasst, der aus der Gruppe bestehend aus einem Alkohol, einem Keton oder einer Carbonsäure in jeder Kombination derselben ausgewählt wird, wobei bei diesem Schritt mindestens ein Teil des restlichen, in der Luft befindlichen CO₂ in der Kultur gelöst wird; und Bestrahlung dieser Kultur mit einer Lichtstrahlung, die multifrequent ist und 40 bis 60 % Strahlung mit einer Wellenlänge zwischen 400 und 475 nm und einer Stärke zwischen 20 W/cm² und 38 W/cm² besitzt,
sodass die Fotosynthese in diesen Mikroorganismen gefördert wird, wodurch eine Verringerung des CO₂-Gehalts der Kultur durch Absorption und/oder Verdauung dieses CO₂ in den Mikroorganismen erreicht und O₂ erzeugt wird.

2. Verfahren nach Anspruch 1, wobei der organische Inhibitor aus Schritt c) Ethanol, Aceton, Propansäure oder Pentansäure in jeder Kombination derselben ist.

3. Verfahren nach Anspruch 2, wobei der organische Inhibitor aus Schritt c) Aceton ist.

4. Verfahren nach Anspruch 1, wobei die auf die Mikroorganismen aufgebrachte Lichtstrahlung ca. 50 % der Strahlung mit einer Wellenlänge zwischen 400 und 475 nm und einer Stärke zwischen 20 w/cm² und 38 W/cm² beinhaltet.

5. Verfahren nach einem der Ansprüche 1 bis 4, wobei beim Schritt c) die Mikroorganismen jede Minute während 3 Sekunden weiterhin mit einer zusätzlichen Lichtstrahlung bestrahlt werden, die eine Wellenlänge von ca. 200 nm und eine Stärke zwischen 5 und 15 W/cm² besitzt, ohne eine Energie von 3·10⁶ eV pro Photon zu überschreiten.

6. Verfahren nach einem der vorstehenden Ansprüche, wobei die Verweilzeit der Mikroorganismen aus dem Schritt c) 4 bis 6 Tage beträgt und alle 5 Tage ein Prozentsatz daraus entfernt wird, der von 10 % bis 40 % der Mikroorganismen variiert.

7. Verfahren nach einem der vorstehenden Ansprüche, wobei anschließend an den Schritt c), der folgende zusätzliche Schritt angewandt wird:
d) maximaler Erzeugungsschritt von O₂, wobei die Kultur aus dem Schritt c) entlang eines Kreislaufs geleitet wird, in dem sie einer aufeinanderfolgenden Reihe von Drücken im Bereich zwischen 0,01 atm und 5,5 atm und einer Bestrahlung mit einer Reihe von Frequenzen des Lichtspektrums ausgesetzt wird, die ebenfalls aufeinanderfolgend Violett, Blau, Grün, Gelb, Orange und Rot durchlaufen und wieder zu Violett zurückkehren, wobei die Bestrahlung eine Stärke zwischen 5 und 50 W/cm² besitzt, wodurch eine zusätzliche Verringerung des CO₂-Gehalts in der Kultur durch Absorption und/oder Verdauung dieses CO₂ in den Mikroorganismen und eine Erzeugung von O₂ verursacht wird.

8. Verfahren nach Anspruch 7, wobei die Kultur der folgenden Lichtstrahlungssequenz ausgesetzt wird:
- ein erster Abschnitt, wobei die Kultur mit einer Lichtstrahlung mit Frequenzen zwischen 400 und 520 nm bei einer Bestrahlungsstärke zwischen 30 und 50 W/cm² bestrahlt und einem Druck zwischen 1,8 und 5,5 atm ausgesetzt wird, wobei diese während eines Zeitraums zwischen 10 Minuten und 24 Stunden darin verbleibt;
- ein zweiter Abschnitt, wobei die Kultur mit einer Lichtstrahlung mit Frequenzen zwischen 521 und 580 nm bei einer Bestrahlungsstärke zwischen 10 und 20 W/cm² bestrahlt und einem Druck zwischen 1,0 und 1,79 atm ausgesetzt wird, wobei diese während eines Zeitraums zwischen 3 Minuten und 24 Stunden darin verbleibt;
- ein dritter Abschnitt, wobei die Kultur mit einer Lichtstrahlung mit Frequenzen zwischen 581 und 620 nm bei einer Bestrahlungsstärke zwischen 21 und 31 W/cm² bestrahlt und einem Druck zwischen 0,5 und 1,25 atm ausgesetzt wird, wobei diese während eines Zeitraums zwischen 3 Minuten und 24 Stunden darin verbleibt;
- ein vierter Abschnitt, wobei die Kultur mit einer Lichtstrahlung mit Frequenzen zwischen 621 und 750 nm bei einer Bestrahlungsstärke zwischen 30 und 5 W/cm² bestrahlt und einem Druck zwischen 0,01 und 1,249 atm ausgesetzt wird, wobei diese während eines Zeitraums zwischen 1 Minute und 24 Stunden darin verbleibt.

9. Verfahren nach Anspruch 8, wobei die Kultur zwischen jedem Abschnitt und dem folgenden Abschnitt einem Dunkelheitszeitraum ausgesetzt wird, wobei sie nicht bestrahlt wird.

10. Verfahren nach einem der vorstehenden Ansprüche 8 oder 9, wobei die Bestrahlungssequenz in Fotoperioden von 14 bis 18 Stunden pro Tag auf die Kultur aufgebracht wird.

11. Verfahren nach einem der vorstehenden Ansprüche, wobei jede bestimmte Anzahl von Tagen mindestens ein Teil der Mikroorganismen aus dem Kreislauf zur maximalen Erzeugung von O₂ entfernt und zu einem zusätzlichen Schritt übertragen wird, bestehend aus:
e) Methanisierung, wobei die Mikroorganismen mittels eines Verfahrens fermentativer Oxidation deaktiviert werden, wodurch eine deaktivierte Biomasse gemischt mit Wasser und ein Biogas umfassend CO₂ und Methan erhalten werden.

12. Verfahren nach Anspruch 11, wobei die fermentative Oxidation unter Verwendung eines Induktors durchgeführt wird, der den Prozess beschleunigt, wobei dieser Induktor Glyzerin ist.

13. System zur Reinigung kontaminierter, CO₂-haltiger Luft durch Mikroorganismen, das die folgenden Elemente umfasst:
a) Empfangssystem (1), das die CO₂-haltige Luft empfängt, die aus einer Quelle kontaminierter Luft stammt;
b) System von Platten (2), die Calcium- und/oder Magnesiumsalze enthalten und dazu dienen, einen Teil des CO₂ der Luft in der Form von Calcium- und/oder Magnesiumkarbonaten zu binden;
**dadurch gekennzeichnet, dass** das System weiterhin Folgendes umfasst:
c) Behälter (3) zur Fermentation der aus den vorherigen Schritten stammenden Luft, die dazu dienen, eine Kultur zu enthalten, die eine Familie aus Mikroorganismen umfasst, die Mikroalgen der Klassen Chlorophyceae, Cyanophyceae, Cryptophyceae, Diatomaceae und/oder Sporen von Braunalgen der Ordnung Laminariales, Rotalgen der Ordnung Gigartinales oder Grünalgen der Ordnung Ulvales in jeder Kombination derselben sowie mindestens einen organischen Inhibitor umfasst, der aus der Gruppe bestehend aus einem Alkohol, einem Keton oder einer Carbonsäure in jeder Kombination derselben ausgewählt wird,
wobei diese Behälter weiterhin eine Lichtstrahlungsquelle umfassen, die dazu in der Lage ist, die Kultur mit einer Lichtstrahlung des sichtbaren Lichtspektrums zu bestrahlen, die multifrequent ist und 40 bis 60 % Strahlung mit einer Wellenlänge zwischen 400 und 475 nm und einer Stärke zwischen 20 W/cm² und 38 W/cm² besitzt.

14. System nach Anspruch 13, das weiterhin einen Kreislauf (4) zur maximalen Erzeugung von O₂ umfasst, der eine Rohrleitungsbaugruppe zum Hindurchleiten der Kultur aus den Fermentierungsbehältern (3) beinhaltet, wobei die Rohrleitung Rohre mit unterschiedlichen Durchmessern im Bereich zwischen 25 und 750 mm umfasst, die in der Lage sind, die Kultur einer bestimmten Sequenz von Drücken zwischen 0,01 atm und 5,5 atm zu unterziehen, sowie eine Lichtstrahlungsquelle, die in der Lage sind, die Kultur gleichzeitig mit einer bestimmten Sequenz von Frequenzen des Lichtspektrums zu bestrahlen, die Violett, Blau, Grün, Gelb, Orange und Rot durchlaufen und wieder zu Violett zurückkehren, mit einer Bestrahlungsstärke zwischen 5 und 50 W/cm².

15. System nach einem der vorstehenden Ansprüche 13 bis 14, das ferner Methanisierungsbehälter (5) umfasst, die dazu dienen, die Mikroorganismen mittels eines Verfahrens fermentativer Oxidation zu deaktivieren, wodurch eine deaktivierte Biomasse gemischt mit Wasser und ein Biogas umfassend CO₂ und Methan erhalten werden.

## Revendications

1. Procédé pour la purification d'air pollué contenant du CO₂ au moyen de microorganismes, comprenant les étapes de :
a) réception d'air contenant du CO₂ provenant d'une source d'air pollué ;
b) catalyse physique, où l'on fait passer l'air contenant du CO₂ au travers de plaques comprenant des sels de calcium et/ou de magnésium, sur lesquelles une partie du CO₂ de l'air se fixe sous la forme de carbonates de calcium et/ou de magnésium ;
**caractérisé en ce que** le procédé comprend en outre les étapes de :
c) fermentation, où l'on fait passer l'air résultant de l'étape b) au travers d'une culture comprenant une famille biologique de microorganismes qui comprend des microalgues des classes chlorophycées, cyanophycées, cryptophycées, diatomées, et/ou spores d'algues qui sont des Laminariales brunes, rouges de l'ordre des Gigartinales ou vertes de l'ordre des Ulvales, dans n'importe laquelle de leurs combinaisons, ainsi qu'au moins un inhibiteur organique sélectionné dans le groupe composé d'un alcool, d'un cétone ou d'un acide carboxylique dans n'importe laquelle de leurs combinaisons, étape dans laquelle au moins une partie du CO₂ restant dans l'air est dissous dans la culture ; et exposition à un rayonnement de ladite culture avec un rayonnement lumineux qui est à multifréquence et comporte de 40 à 60% de rayonnement ayant une longueur d'onde comprise entre 400 et 475 nm et une intensité comprise entre 20 W/cm² et 38 W/cm²,
de façon que la photosynthèse soit lancée dans lesdits microorganismes, provoque une réduction de la teneur en CO₂ dans la culture par absorption et/ou digestion dudit CO₂ dans les microorganismes et produise de l'O₂.

2. Procédé selon la revendication 1 où l'inhibiteur organique de l'étape c) est de l'éthanol, de l'acétone, de l'acide propionique ou de l'acide pentanoïque, dans n'importe laquelle de leurs combinaisons.

3. Procédé selon la revendication 2 où l'inhibiteur organique de l'étape c) est de l'acétone.

4. Procédé selon la revendication 1 où le rayonnement lumineux appliqué aux microorganismes comporte environ 50% de rayonnement ayant une longueur d'onde comprise entre 400 et 475 nm et une intensité comprise entre 20 W/cm² et 38 W/cm².

5. Procédé selon n'importe laquelle des revendications 1 à 4 où dans l'étape c) les microorganismes sont de plus exposés à un rayonnement durant 3 secondes à chaque minute avec un rayonnement lumineux supplémentaire ayant une longueur d'onde d'environ 200 nm et une intensité comprise entre 5 et 15 W/cm², sans dépasser une énergie de 3·10⁶ eV par photon.

6. Procédé selon n'importe laquelle des revendications précédentes où le temps de maintien des microorganismes dans l'étape c) va de 4 à 6 jours, et où, tous les 5 jours, un pourcentage variant de 10% à 40% des microorganismes en est retiré.

7. Procédé selon n'importe laquelle des revendications précédentes où, après l'étape c), l'étape supplémentaire suivante est appliquée :
d) étape de production maximale d'O₂ où l'on fait passer la culture résultant de l'étape c) le long d'un circuit où elle est soumise à une série de pressions successives allant de 0,01 atm à 5,5 atm et à une exposition à un rayonnement avec une série de fréquences du spectre lumineux passant du violet au bleu, vert, jaune, orange et rouge, pour revenir au violet, successivement également, le rayonnement ayant une intensité comprise entre 5 et 50 W/cm², cela provoquant une réduction supplémentaire de la teneur en CO₂ dans la culture par absorption et/ou digestion dudit CO₂ dans les microorganismes et produisant de l'O₂.

8. Procédé selon la revendication 7 où la culture est soumise à la séquence suivante de rayonnement lumineux :
- une première section où la culture est exposée à un rayonnement avec un rayonnement lumineux ayant des fréquences comprises entre 400 et 520 nm à une intensité de rayonnement comprise entre 30 et 50 W/cm² et est soumise à une pression allant de 1,8 à 5,5 atm, restant dans ladite section durant une période comprise entre 10 minutes et 24 heures ;
- une deuxième section où la culture est exposée à un rayonnement avec un rayonnement lumineux ayant des fréquences comprises entre 521 et 580 nm à une intensité de rayonnement comprise entre 10 et 20 W/cm² et est soumise à une pression allant de 1,0 à 1,79 atm, restant dans ladite section durant une période comprise entre 3 minutes et 24 heures ;
- une troisième section où la culture est exposée à un rayonnement avec un rayonnement lumineux ayant des fréquences comprises entre 581 et 620 nm à une intensité de rayonnement comprise entre 21 et 31 W/cm² et est soumise à une pression allant de 0,5 à 1,25 atm, restant dans ladite section durant une période comprise entre 3 minutes et 24 heures ;
- une quatrième section où la culture est exposée à un rayonnement avec un rayonnement lumineux ayant des fréquences comprises entre 621 et 750 nm à une intensité de rayonnement comprise entre 30 et 5 W/cm² et est soumise à une pression allant de 0,01 à 1,249 atm, restant dans ladite section durant une période comprise entre 1 minute et 24 heures.

9. Procédé selon la revendication 8 où entre chaque section et celle qui suit, la culture est soumise à une période d'obscurité durant laquelle elle n'est pas exposée à un rayonnement.

10. Procédé selon n'importe laquelle des revendications 8 ou 9 qui précèdent, où la séquence d'expositions à un rayonnement est appliquée à la culture par photopériodes allant de 14 à 18 heures par jour.

11. Procédé selon n'importe laquelle des revendications précédentes où chaque nombre de jours particulier au moins une partie des microorganismes est retirée du circuit de production maximale d'O₂ et transférée vers une étape supplémentaire de :
e) méthanisation, où les microorganismes sont désactivés au moyen d'une procédure d'oxydation de fermentation, par laquelle on obtient une biomasse désactivée mélangée avec de l'eau et un biogaz comprenant du CO₂ et du méthane.

12. Procédé selon la revendication 11 où l'oxydation de fermentation est réalisée en utilisant un inducteur accélérant le procédé, ledit inducteur étant du glycérol.

13. Système pour purifier de l'air pollué contenant du CO₂ au moyen de microorganismes comprenant les éléments suivants :
a) systèmes de réception (1) recevant de l'air contenant du CO₂ provenant d'une source d'air pollué ;
b) système de plaques (2) contenant des sels de calcium et/ou de magnésium destinés à fixer une partie du CO₂ de l'air sous la forme de carbonates de calcium et/ou de magnésium ;
**caractérisé en ce que** le système comprend en outre :
c) des cuves (3) pour la fermentation de l'air provenant de l'étape précédente destinées à contenir une culture comprenant une famille de microorganismes qui comprend des microalgues des classes chlorophycées, cyanophycées, cryptophycées, diatomées, et/ou des spores d'algues qui sont des Laminariales brunes, rouges de l'ordre des Gigartinales ou vertes de l'ordre des Ulvales, dans n'importe laquelle de leurs combinaisons, ainsi qu'au moins un inhibiteur organique sélectionné dans le groupe composé d'un alcool, d'un cétone ou d'un acide carboxylique dans n'importe laquelle de leurs combinaisons,
où ces cuves comprennent en outre une source de rayonnement lumineux capable d'exposer la culture à un rayonnement avec un rayonnement lumineux du spectre lumineux visible qui est à multifréquence et comporte de 40 à 60% de rayonnement ayant une longueur d'onde comprise entre 400 et 475 nm et une intensité comprise entre 20 W/cm² et 38 W/cm².

14. Système selon la revendication 13 comprenant en outre un circuit (4) de production maximale d'O2, comprenant un assemblage de tuyaux destinés au passage dans ces derniers de la culture provenant des cuves de fermentation (3), cet assemblage de tuyaux comprend des tuyaux de différents diamètres compris entre 25 et 750 mm capables de soumettre la culture à une séquence particulière de pressions allant de 0,01 atm à 5,5 atm, et une source de rayonnement lumineux capable d'exposer la culture à un rayonnement avec une séquence particulière de fréquences du spectre lumineux passant du violet au bleu, vert, jaune, orange et rouge, pour revenir au violet, avec une intensité de rayonnement comprise entre 5 et 50 W/cm², simultanément.

15. Système selon n'importe laquelle des revendications 13 ou 14 qui précèdent, comprenant en outre des cuves de méthanisation (5) destinées à désactiver les microorganismes au moyen d'une procédure d'oxydation de fermentation qui y est obtenue à partir d'une biomasse désactivée mélangée avec de l'eau et un biogaz comprenant du CO₂ et du méthane.
